# EUROPEAN PATENT APPLICATION

(11) **EP 4 529 915 A1**
(43) Date of publication of application: **02.04.2025**
(21) Application number: 23200263.4
(22) Date of filing: 28.09.2023
(51) Int. Cl.: A61K 9/00, A61K 9/08, A61K 9/127, A61K 31/175, A61K 47/40, A61K 47/69, A61P 35/00

(54) **LIPOSOMAL PREPARATIONS FOR USE IN TREATING CANCER**

(71) Applicant: DG-Nika AG, 6374 Nidwalden (CH)
(72) Inventor: GUBERNATOR, Jerzy, 6374 NIDWALDEN (CH); KOPPER, Helga, 6374 NIDWALDEN (CH)
(74) Representative: Schweiger, Martin

(57) **Abstract**

Drug delivery solutions are provided for encapsulation within the aqueous solution core of liposomes for use in treating cancers and other tumors, the drug delivery solutions comprising an aqueous solution comprising lomustine or another drug and cyclodextrins comprising methyl-β-CD or hydroxy propyl-β-CD.

## Description

### Field of the Invention

The present invention relates to a drug delivery solution for encapsulation within the aqueous core of liposomes for use in treating cancers and other tumors, the drug delivery solution comprising an aqueous solution comprising at least one drug and cyclodextrins.

### Background

The following discussion of the background art is intended to facilitate an understanding of the present invention only. It should be appreciated that the discussion is not an acknowledgement or admission that any of the material referred to was part of the common general knowledge as at the priority date of the application.

Gliomas are the most common malignant tumors of the central nervous system. They arise from glial cells and are most often localized in the vicinity of the temporal, occipital, frontal and parietal lobes, and usually not in the cerebellum. Depending on the malignancy, they can be grade 1 through to grade 4. There are several types of gliomas, some of which are more malignant than others. Glioblastomas (GBM) are highly malignant and invasive tumors (grade 4 gliomas) with very low survival rates, despite significant improvements in the treatment options for patients with this type of cancer. They can affect people of all ages but are most commonly diagnosed in adults between the ages of 50 and 70. They differ in their location in the brain, the way they grow, and their response to treatment. The lack of consistency between different types of tumor and its different histological origin are a significant obstacle for its subsequent therapy.

Treatment options are severely limited by the blood-brain barrier (BBB) and the blood-cancer-brain barrier (Blood-Tumor Barrier, or BTB), which severely impedes the flow of treatment compounds to the brain. The BBB does not have many of the receptors present in other cells, and in addition, there is no system that could allow the transport of acid hydrolases. Many drugs and therapeutic enzymes have difficulty getting through the BBB because of their high molecular weight and the fact that they are often not hydrophobic, make any transport, intercellular or paracellular, impossible. In this respect, some 900+ of low molecular weight drugs and nearly 100% of all larger drugs produced to date fail to overcome the BBB.

Typically, treatment for GBM is based on surgery, radiotherapy and chemotherapy with temozolomide (TMZ), the strongest therapeutic agent against GBM. Despite the high effectiveness of TMZ, the prognosis of patients is still not good, which results from the innate or acquired resistance of cancer cells to this drug. The average survival of those affected is 15 months, and the proportion of those living 5 years or more is only 10%.

Increasing knowledge in the field of molecular pathology has offered new solutions and better treatments for several cancers. Unfortunately, glioblastoma patients have not yet benefited, even though many experimental drugs have been tested in clinical trials. The current grim prognosis for glioblastoma is at least partly due to the lack of effective drug delivery through the BBB and BTB, and also due to its flexible metabolism.

Lomustine is an alkylating anticancer drug which can efficiently pass through the BBB. It non-specifically mutates the cell cycle of tumor cells at the G1 stage, G2 stage, G2/S, and M stage. Lomustine is able to enhance the sensitivity of tumor cells and induce apoptotic cell death through TRC8-mediated degradation targeting heme oxygenase-1. Current understanding is that lomustine acts by a dual mechanism with the cytotoxic effect involving the inhibition of DNA and RNA synthesis through alkylation, and interference with histidine utilization, thereby upsetting the 1-carbon metabolic transfer process. Lomustine is mainly used in the treatment of brain tumors (including glioblastoma) and leukemia but has also been used in the treatment of Hodgkin's disease, non-Hodgin's lymphomas, lung cancer, malignant melanoma, breast carcinoma, bronchogenic carcinoma, renal cell carcinoma, carcinoma of the GI tract, and various solid tumors.

Conventionally, lomustine is orally administered, although it has very low bioavailability and significant side effects. It is absorbed from the gastro-intestinal tract and is rapidly metabolized into metabolites that have a prolonged plasma half-life ranging from 16 to 48 h. Lomustine and its metabolites cross the BBB and are rapidly transported into cells due to their high lipid solubility. Lomustine is not detectable in cerebral spinal fluid (CSF) but active metabolites can appear in substantial concentrations within 30 min after oral administration of lomustine, and about half the dose of the drug is excreted as metabolites in the urine within 24 hours, but less than 75% within 4 days. About 600 of the cyclohexyl moiety of lomustine is reported to be bound to plasma proteins. The second phase plasma half-life is 1 to 2 days and 15-20% of the metabolites remain in the body 5 days after administration. Prolongation of plasma concentration is thought to reflect a combination of protein binding and enterohepatic circulation of metabolites. Lomustine is distributed among the tissues and in the cerebrospinal fluid, the concentration of metabolites reaches 150% of that in plasma. Biotransformation occurs throughout the body with a half-life of less than 1 hour. Lomustine metabolism also causes toxicity to cells of the digestive system. Thus, while lomustine has great potential as an anticancer drug, all of these factors limit its potential usefulness in treatment of cancers when taken in an oral form.

A potential alternative to oral dosage forms for lomustine and other drugs for use in treating brain tumors is the use of liposomal delivery by injection into a patient. Liposomes can improve the stability and pharmacokinetics and reduce the toxicity of many drugs. Liposomes are oval structures made of a lipid bilayer with a size from about 20 nm for small unilamellar liposomes to even several micrometers for large multilamellar liposomes. Small unilamellar liposomes are good carriers for hydrophobic drugs as well as multilamellar liposomes while large unilamellar liposomes are good for carrying hydrophilic drugs.

However, a considerable difficulty is in the encapsulation of hydrophobic/lipophilic drugs with a low logP parameter of less than 3.5 which includes lomustine. Such substances are characterized by low hydrophobicity/lipophilicity and at the same time they are not soluble in water. Generally, for such drugs, it is possible to use liposomes and encapsulate them in a liposome bilayer. However, this results in a number of disadvantages such as low incorporation efficiency, rapid drug crystallization, difficulty in producing the liposome suspension, and poor drug pharmacokinetics due to very rapid drug release in the bloodstream due to interaction with blood proteins.

It would therefore be of benefit to identify new means for delivery of drugs such as lomustine for use in treating various cancers including glioblastomas and other tumors of the brain.

Throughout the specification unless the context requires otherwise, the word "comprise" or variations such as "comprises" or "comprising", will be understood to imply the inclusion of a stated integer or group of integers but not the exclusion of any other integer or group of integers.

Throughout the specification unless the context requires otherwise, the word "include" or variations such as "includes" or "including", will be understood to imply the inclusion of a stated integer or group of integers but not the exclusion of any other integer or group of integers.

### Summary of Invention

According to a first aspect of the invention, there is provided a drug delivery solution for encapsulation within the aqueous core of liposomes, the drug delivery solution comprising:
an aqueous solution comprising at least one drug and cyclodextrins.

In an embodiment, the aqueous solution comprises water.

In an embodiment, the cyclodextrins comprise methyl-β-CD or hydroxy propyl-β-CD.

In an embodiment, the methyl-β-CD or hydroxy propyl-β-CD comprises between approximately 1 and 45% by weight of the drug delivery solution. The methyl-β-CD or hydroxy propyl-β-CD preferably comprises between approximately 5 and 35% by weight of the drug delivery solution. More preferably, the methyl-β-CD comprises approximately 25% by weight of the drug delivery solution.

In an embodiment, the drug is mixed into and dissolves with the cyclodextrins in aqueous solution.

In an embodiment, the drug is mixed into and dissolves with the cyclodextrins in aqueous solution at room temperature.

In an embodiment, the at least one drug comprises lomustine.

In an embodiment, lomustine is added to methyl-β-CD and the compounds are mixed in 20 mM citrate buffer pH 4.5 to form the drug delivery solution.

In an embodiment, lomustine is added to hydroxy propyl-β-CD and the compounds are mixed in 20 mM citrate buffer pH 4.5 to form the drug delivery solution.

In an embodiment, the concentration of lomustine in the drug delivery solution is between approximately 1 to 40 mg/ml. The concentration of lomustine in the drug delivery solution is preferably between approximately 10 to 30 mg/ml. More preferably, the concentration of lomustine in the drug delivery solution is approximately 20 mg/ml.

In an embodiment, the amount of lomustine is between approximately 0.025 to 4% of the total weight of the drug delivery solution. The amount of lomustine is preferably between approximately 0.5 to 1.5% of the total weight of the drug delivery solution when the cyclodextrin comprises hydroxy propyl-β-CD. More preferably, the amount of lomustine is between approximately 1 to 3% of the total weight of the drug delivery solution when the cyclodextrin comprises methyl-β-CD.

In an embodiment, lomustine is added to methyl-β-CD and the compounds are mixed in 20 mM citrate buffer pH 4.5 to form the drug delivery solution.

In an embodiment, lomustine is added to hydroxy propyl-β-CD and the compounds are mixed in 20 mM citrate buffer pH 4.5 to form the drug delivery solution.

According to a second aspect of the invention, there is provided a method of forming liposomes comprising:
combining a drug delivery solution as described herein with phospholipids, lipids, sterols to form a liposome forming mixture;
drying to evaporate the liquid from the liposome forming mixture;
rehydrating the liposome forming mixture in water and excipients to form multilamellar liposomes.

In an embodiment, the pH of the aqueous solution core of the liposomes is less than approximately pH 5.5. The pH of the aqueous solution core of the liposomes is preferably between approximately pH 4.0 to 5.0.

In an embodiment, the phospholipids and other lipids comprises between approximately 0.1 to 10% by weight (w/w) of the total mass of liposomes suspension. The phospholipids and other lipids preferably comprises between approximately 1 to 5% by weight (w/w) of the total mass of liposomes suspension. In this respect, the Liposomes can be composed only of phospholipids (with the addition of pegylated or polyglycerolated phospholipids), or of phospholipids (with the addition of pegylated or polyglyceroled phospholipids), or of sterols (cholesterol).

In an embodiment, the main phospholipids comprise one or more selected from the group comprising: sphingomyelins (SM), dipalmitoylphosphatidylcholine (DPPC), distearoylphosphatidylcholine (DSPC), and hydrogenated soybean phospholipids (HSPC).

In an embodiment, the auxiliary phospholipids and lipids comprise one or more selected from the group comprising: 1,2-Dipalmitoyl-sn-glycero-3-phosphoglycerol, sodium salt (DPPG), 1,2-Distearoyl-sn-glycero-3-phosphoglycerol, sodium salt (DSPG), pegylated, polyglycerylated phospholipids, and/or sterols containing PEG covalently attached to the lipid molecule (pegylated lipids) having a molecular weight from between approximately 350 to 6000 Da.

In an embodiment, the non-pegylated auxiliary phospholipids are used in a molar % from between approximately 2.5 to 35 molaro. The non-pegylated auxiliary phospholipids are preferably used in a molar % from between approximately 15 to 25 molaro. More preferably, the non-pegylated auxiliary phospholipids are used in a molar % of approximately 20 molaro.

In an embodiment, the pegylated lipids are used in a molar % in relation to other lipids from between approximately 1 to 10 molaro. The pegylated lipids are preferably used in a molar % from between approximately 2 to 5 molar%. More preferably, the pegylated lipids are used in a molar % of approximately 5 molaro.

In an embodiment, the sterols (cholesterol) are used in a molar % from between approximately 0 to 50 molaro. The sterols (cholesterol) are preferably used in a molar % from between approximately 20 to 30 molaro.

In an embodiment, the excipients comprise one or more from the group selected from: amino acids, vitamins, organic acids and their salts, saccharose, and targeting moieties used for liposome targeting. The amino acids preferably comprise glycine and/or histidine. The vitamins preferably comprise nicotinic acid amide. Targeting moieties preferably comprise one or more of transferrin, folic acid, and peptides.

According to a third aspect of the invention, there is provided a method of forming liposomes comprising:
dissolving a lipid composition in an organic solvent and then drying the dissolved lipids;
resuspending the lipids in a drug delivery solution as described herein to form multilamellar liposomes;
extruding the multicellular liposomes to form large unilamellar liposomes; and
removing non-encapsulated drug by gel filtration.

In an embodiment, the lipid composition comprises HSPC/DSPE-PEG 2000 (95:5, mol/mol).

In an embodiment, the lipids composition comprises HSPC/DSPG/DSPE-PEG 2000 (75:20:5, mol/mol).

In an embodiment, the lipids composition comprises HSPC/Chol/DSPE-PEG 2000 (65:30:5, mol/mol).

In an embodiment, the lipids composition comprises SM/DSPE-PEG 2000 (95:5, mol/mol).

In an embodiment, the lipids composition comprises SM/Chol (45:55, mol/mol).

In an embodiment, the organic solvent comprises one or more selected from the group comprising: cyclohexane, methanol, and ethanol.

In an embodiment, the drying comprises freeze drying. The freeze drying preferably comprises using liquid nitrogen.

In an embodiment, the drying comprises evaporation. The evaporation preferably uses a rotary evaporator and vacuum.

In an embodiment, the lipids are resuspended in the drug delivery solution at approximately 64 °C.

In an embodiment, the multicellular liposomes are extruded through a 400 nm and then 100 nm polycarbonate filter using a thermobarrel extruder.

In an embodiment, the non-encapsulated drug is removed by gel filtration using a Sephadex G50 fine column equilibrated with 10% saccharose and 20 mM citrate buffer pH 4.5.

In an embodiment, the size of the unilamellar liposomes was between approximately 40 to 250 nm, more preferably between approximately 90 to 130 nm.

In an embodiment, the drug encapsulation efficiency of the method was between approximately 3.0 to 6.5%.

In an embodiment, liposomes produced by a method as described herein are lyophilised and then rehydrated in water. The liposomes are preferably lyophilised by freeze-drying at approximately 40 °C for 24 h.

In an embodiment, the rehydrated liposomes are between approximately 105 to 125 nm. The rehydrated liposomes are preferably approximately 117 nm.

In an embodiment, the rehydrated liposomes have a PDI Index of between approximately 0.1 to 0.2. The rehydrated liposomes preferably have a PDI Index of approximately 0.132.

In an embodiment, the liposomes have an encapsulation efficiency of between approximately 3-7%, more preferably between approximately 3.9-6.1%. The liposomes have an encapsulation efficiency of between approximately 4-6%.

According to a fourth aspect of the invention, there is provided liposomes formed by a method as described herein.

In an embodiment, the present invention provides a method of treating a subject in need thereof with liposomes as described herein.

In an embodiment, the present invention provides liposomes formed by a method as described herein for treating cancer.

In an embodiment, the present invention provides a method of treating cancer using liposomes formed by a method as described herein.

In an embodiment, the present invention provides use of liposomes as described herein for administration to a subject in need thereof in the treatment of cancer.

In an embodiment, the present invention provides use of lomustine in the manufacture of a medicament comprising liposomes as described herein for the therapeutic treatment of cancer.

In an embodiment, treating the subject comprises injecting the subject with the liposomes. The subject is preferably injected subcutaneously.

In an embodiment, the subject is treated for cancer or a tumor.

In an embodiment, the subject is treated for one or more selected from the group comprising: brain tumors, gliomas, leukemia, lymphomas, Hodgkin's disease, non-Hodgin's lymphomas, lung cancer, blood cancers, malignant melanoma, breast carcinoma, bronchogenic carcinoma, renal cell carcinoma, carcinoma of the GI tract, and various solid tumors. The subject is preferably treated for glioblastoma.

While lomustine is a valuable alkylating drug that crosses the BBB and can be used in the treatment of glioblastoma and in the treatment of other solid and blood cancers, its use is currently limited to oral administration. This reduces the bioavailability of the drug and to a large extent leads to the appearance of biotransformation products (metabolites) of the drug in the bloodstream, and not the parent drug.

There was therefore scope to develop an injectable form of lomustine in a liposome or other nanocarrier delivery system, but which addressed the common disadvantages of using liposomes, including: low incorporation efficiency, rapid drug crystallization, difficult production of the liposome suspension, and poor drug pharmacokinetics due to very rapid drug release in the bloodstream due to interaction with blood proteins. The use of liposomes can further increase the bioavailability of the drug, increase the penetration of the BBB, and reduce side effects by reducing the dose of the drug required. In the case of liposomes, a relatively low logP limits the effective and permanent encapsulation of the drug in the liposome membrane. For this reason, a way to encapsulate the drug in the water space of liposomes was sought after finding a method of preparing a solution of the drug in water that can be encapsulated in this way.

Inventors found that when encapsulating lomustine in liposomes, they were able to produce liposomes with a drug incorporated in the lipid bilayer with a relatively high level of efficiency. However, they continually observed a rapid crystallisation or leakage of the drug from the liposomes during storage or after dilution or mixing with human plasma.

However, they made an unexpected discovery that a methyl-β-CD (methyl-β-cyclodextrin) solution was able to dissolve lomustine crystals to the aqueous phase within a few seconds. They determined that the mechanism of this process is mostly related to co-dissolution rather than the formation of an inclusion complex as is often the case. In this respect, the formation of an inclusion complex usually takes from a few to several hours and requires constant mixing, heating and the use of a co-solvent such as ethanol or methanol. But when using a 25% concentrated methyl-β-CD solution, the dissolution process unexpectedly took only a few seconds at room temperature (R.T.) and resulted in a solution of lomustine with a concentration of at least 20 mg/ml. The final lomustine concentration was found to be suitable for passive encapsulation of the drug in the aqueous space of liposomes of appropriate composition avoiding the previous problems of crystallisation or leakage, and in addition, allowed several methods of drug encapsulation to be used.

### Brief Description of Drawings

In order to provide a better understanding, embodiments of the present invention will be described, by way of example only, with reference to the accompanying drawings, in which:

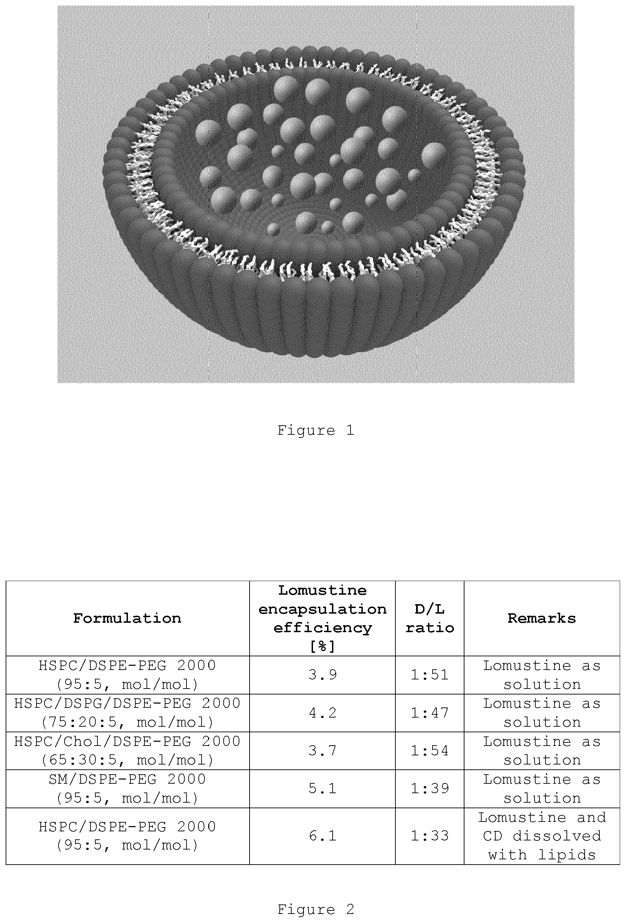

- Figure 1: Illustration showing the localisation of lomustine solution/inclusion complexes with cyclodextrins within unilamellar liposomes as shown in Examples 3 to 12.
- Figure 2: Table showing the encapsulation efficiencies of lomustine within liposomes and drug to lipid ratio in the preparations depending on the composition of the liposomes and the preparation method.

### Description of Preferred Embodiments

In order to provide a more precise understanding of the subject matter of the invention, features of the invention will now be discussed with reference to the following preferred embodiment or embodiments.

### Transport Across the Blood Brain Barrier

The transport of substances is significantly limited in the BBB. Paracellular transport occurs, but under very restrictive conditions. Only small, hydrophobic and to some extend positively charged molecules can squeeze between the endothelial cells from the blood to the cerebrospinal fluid, or vice versa. Other molecules need specific systems to pass through this barrier and there are six main mechanisms of this transport.

The first is simple diffusion dependent on the gradient of molecules. Small, hydrophobic and positively charged molecules can pass through the cell membrane and through endothelial cells to be transported to the cerebrospinal fluid. Their mass must not exceed 400 to 600 Da and must not contain more than eight hydrogen bonds. In addition, the more hydrophobic a molecule is, the easier this process occurs. This is how respiratory gases from the bloodstream can enter the brain. Additionally, this method can also be used to transport drugs. All other molecules that do not meet the above criteria must use specific systems to pass through the barrier. Paracellular movement of molecules is possible and also depends on the gradient of the substance. However, it is closely related to the amount of tight junctions (TJs) in endothelial cells, their size, which cannot exceed 180 Da, and their hydrophobicity.

A second mechanism is facilitated diffusion. It can be divided into two types - inside the cell (uptake) and outside it (efflux). Transport into the cell is possible thanks to a family of carrier proteins (solute carriers - SLC). SLC proteins also allow bidirectional transport and function as efflux messengers, but this is less common. These proteins follow a concentration gradient and can carry large molecules across the barrier. They are responsible for supplying one of the most important substances to the central nervous system - including glucose, vitamins, amino acids, ions, neurotransmitters, fatty acids and other sugars. They do not need ATP to function and this is what makes them different from ABC transporters.

A third such mechanism is transport facilitated against the concentration gradient, which is made possible by the ATP-binding cassette (ABC) family of transport proteins. It is mainly thanks to them that transport outside the cell is possible. It is worth noting that the transport of drugs to the brain is mainly due to the family of SLC proteins, but this is also associated with many limiting factors. On the other hand, ABC transporters are most often responsible for the removal of metabolites of drug action or themselves from this system. It is mainly this family of proteins that is involved in the drainage of substances and molecules from the BBB. They have a wide range of applications, especially for the transfer of large, fat-soluble substances that have many hydrogen bonds and oxygen and nitrogen atoms in their structure. This transport takes place thanks to the hydrolysis of ATP, thanks to which it can take place against the concentration gradient. The main ABC transporters in the brain are ABCB1 and ABCG2.

The fourth is receptor-mediated transcytosis (RTM). It is RTM which mainly enables the transport of important macromolecules needed for the proper functioning of the brain through the blood-brain barrier. Large proteins, antibodies, or peptide ligands bind to RTM receptors and are thus used to deliver macromolecules, including drugs, to the brain. These receptors are located on endothelial cells on the side of the bloodstream, and this system uses endocytosis, vesicular transport and exocytosis to transport substances from the apical part of the cell to its basal part.

The fifth is adsorptive-mediated transcytosis (AMT), which is a method of transporting macromolecules and highly charged nanoparticles across the BBB. The ATM technique uses significant electrostatic interactions between positively charged drug transporters and negatively charged microdomains on the endothelial cell membrane. However, this is not a specific procedure and may contribute to the accumulation of drugs in other organs.

The sixth and last of these mechanisms is the transport of leukocytes through the endothelial cells of the brain, also known as diapedesis. They can directly pass through endothelial cells without disturbing the tight junctions that occur between them. They can both pass between them and through their membrane and cytoplasm. After the leukocytes have been transported through the endothelium, they become immune cells of the brain. Their flow through the barrier increases with the appearance of inflammation.

The BBB does not have many of the receptors present in other cells, and in addition there is no system that could allow the transport of acid hydrolases. The high molecular weight of the compounds and the fact that they are not hydrophobic make any transport - intercellular or paracellular - impossible. Therefore, many drugs and therapeutic enzymes have difficulty getting through the barrier. Research suggests that over 90% of low molecular weight drugs and nearly 100% of all larger drugs fail to overcome the BBB.

### Liposomes

Liposomes can be made of various components, but they must be amphiphilic substances with a suitable molecular shape. The most common components of liposomes are natural phospholipids such as natural egg lecithin, natural soybean lectin, natural sphingomyelin. Semi-synthetic phospholipids, which include DMPC, DPPC, HSPC, DSPC and others, play an important role in the pharmaceutical industry. An important component of liposomes is cholesterol, which is added to the composition of liposomes in an amount of 10 to 50 mol% in order to increase the packing parameter of liposome membranes made of natural phospholipids and reduce the packing parameter in the case of liposomes made of semi-synthetic phospholipids containing saturated fatty acids. In addition to reducing the packing parameter, cholesterol abolishes the phase transition and packing defects of liposome membranes leading to their aggregation.

An important component of liposome membranes is polymers that make them invisible to the immune system. The most commonly used polymers attached to DSPE are polyethylene glycol with a weight of 350 to 5000 Daltons. The molar addition of pegylated phospholipids is most often from 1 to 5 mol% and in some cases may even be 10 mol%. In addition to polyethylene glycol, another polymer such as heparin, hyaluronic acid, polyglycerol or polyvinyl alcohol can be used as a component of liposomes that increase their stability. Potential components that may provide stability to liposomes include albumin, which may be covalently attached to liposomes surface.

One of the most important applications of liposomes is in the pharmaceutical industry. At the moment, there are several liposome preparations on the market containing anticancer drugs, antifungal drugs and substances that sensitize after irradiation. In addition to preparations containing single drugs, there is also a preparation on the market, Vyxeos, containing two anticancer drugs acting in synergy.

### Lomustine and Liposome Preparations

As described above, lomustine is a valuable alkylating drug that crosses the BBB, can be used in the treatment of GBM and in the treatment of other solid and blood cancers. Its use was currently limited to oral administration, which reduces the bioavailability of the drug and to a large extent leads to the appearance of biotransformation products of the drug in the bloodstream, and not the parent drug.

Inventors undertook to develop an injectable form of this drug in the form of liposomes or other nanocarriers. In the case of liposomes, a relatively low logP limits the effective and permanent encapsulation of the drug in the liposome membrane. For this reason, a way to encapsulate the drug in the water space of liposomes (the 'aqueous solution core') was sought after identifying a method of preparing a solution of the drug in water that can be encapsulated in this way.

The present invention provides a method of preparing a liposomal form of lomustine by (i) producing its aqueous solution of high concentration and (ii) then encapsulating it in the aqueous space of liposomes. Thanks to the use of selected cyclodextrins it is possible to obtain a high concentration of lomustine in water of ~20 mg / ml. The normal concentration of lomustine is not more than 0.11 mg/ml when no additives are used. This method makes it possible to encapsulate lomustine in the water space of liposomes in the form of its solution in water or/and inclusion complex with cyclodextrins, or to obtain the effect of co-dissolving lomustine and cyclodextrin in an aqueous solution. The main advantage of this solution is to limit the crystallization and escape of the drug from the liposomes by transferring it to an aqueous solution using not organic solvents such as ethanol, but various organic substances that can be administered intravenously without harming the patient and not damaging the liposomes as is the case with high concentrations of ethanol needed to obtain a high concentration of lomustine in water.

The stability of the liposomes can be further extended by lyophilizing the liposome suspension so as to obtain a dry form of the liposomes in order to avoid the degradation process of lomustine in water. The pH of the internal solution must be kept below pH=5.5, preferably in the range of 4.0 to 5.0. In this case, the cyclodextrins inside the liposomes are a factor that protects the liposomes against aggregation and loss of the encapsulated substance.

During the research on the encapsulation of lomustine in liposomes, inventors developed liposomes with a drug incorporated in the lipid bilayer of liposomes with a relatively high efficiency of the process, but at the same time they observed a rapid crystallisation or leakage of the drug from the liposomes after dilution or mixing with human plasma.

For this reason, they were looking for a way to effectively encapsulate the drug in the aqueous space of liposomes as a real solution. While searching for substances that could potentially enable the transfer of lomustine from its crystals to the aqueous phase, inventors unexpectedly discovered that a methyl-β-CD solution dissolved lomustine within a few seconds. It allowed them to obtain a solution of lomustine with a concentration of at least 20 mg/ml at R.T. if 25% solution of the concentration of methyl-β-CD is used. The final lomustine concentration was suitable for passive encapsulation of the drug in the aqueous space of liposomes of appropriate composition. With the use of cyclodextrins, it was possible to obtain a very high concentration of lomustine in an aqueous solution, which allows it to passively encapsulate its solution in the water space of liposomes. The use of one of the cyclodextrins makes it possible to develop a new method of drug encapsulation other than the one used so far, which uses the amphiphilic properties of the drug by incorporating it in the liposome membrane. The co-dissolution approach of the drug in the cyclodextrin solution allows for a high drug/lipid ratio and thus enables the practical use of lomustine in liposomal form.

In a methyl-β-CD solution comprising lomustine of the invention, the methyl-β-CD concentration in solution is preferably between approximately 1 and 45%.

Various methods of drug encapsulation may be used with the methyl-β-CD solution comprising dissolved lomustine of the invention as discussed below and in the examples as follows.

In an embodiment, lomustine may be prepared as a solution in water containing methyl-β-CD. In a methyl-β-CD solution comprising lomustine of the invention, the methyl-β-CD concentration in solution is preferably between approximately 1 and 45%. The lomustine concentration is preferably between approximately 1 to 35 mg/ml. The resulting solution can be encapsulated within the water space or aqueous solution core of liposomes.

In an embodiment, lomustine may be prepared as a solution in water containing hydroxy propyl-β-CD. In a hydroxy propyl-β-CD solution comprising lomustine of the invention, the hydroxy propyl-β-CD concentration in solution is preferably between approximately 1 and 45%. The lomustine concentration is preferably between approximately 1 to 35 mg/ml. The resulting solution can be encapsulated within the water space or aqueous solution core of liposomes.

In an embodiment, lomustine can be prepared as a solution in ethanol with phospholipids, sterols and other lipids used for liposomes preparation.

In an embodiment, lomustine can be encapsulated within liposomes as solution inside the internal space or aqueous solution core of liposomes.

In an embodiment, a lomustine solution in ethanol with methyl-β-CD, phospholipids, other lipids used for liposomes preparation, or/and sterols, is evaporated and the resulting drug/lipid mass is hydrated by the addition of water containing selected excipients to form multilamellar liposomes.

In an embodiment, a lomustine solution in ethanol with hydroxy propyl-β-CD, phospholipids, and other lipids, or/and sterols, used for liposomes preparation is evaporated and the resulting drug/lipid mass is hydrated by addition of water containing selected excipients to form multilamellar liposomes.

In an embodiment, multilamellar liposomes containing lomustine solution with cyclodextrins are subjected to processes in order to modify their size including, for example, amongst others: extrusion, high pressure homogenisation, and/or sonication.

A preparation according to the invention may comprise a solution of between approximately 0.025 to 3% by weight (w/w)(of the total weight of the preparation) of lomustine, more preferably 1 to 3% by weight of lomustine, and between approximately 5 to 35% by weight of methyl-β-CD in water.

A preparation according to the invention may comprise a solution of between approximately 0.025 to 1.5% by weight of lomustine, more preferably 0.5 to 1.5% by weight of lomustine, and between approximately 5 to 35% by weight of hydroxy propyl-β-CD in water.

A preparation according to the invention as described herein, further comprising a lipid fraction.

The lipid fraction preferably comprises a phospholipid fraction.

The phospholipid and other lipid fraction preferably comprises between approximately 0.1% to 10% w/w, more preferably 1% to 5% w/w, most preferably 5% w/w, of the total mass of liposomal formulation.

The phospholipids preferably comprise sphingomyelins (SM), dipalmitoylphosphatidylcholine (DPPC), distearoylphosphatidylcholine (DSPC), hydrogenated soybean phospholipids (HSPC) and an auxiliary lipids for example 1,2-Dipalmitoyl-sn-glycero-3-phosphoglycerol, sodium salt (DPPG), 1,2-Distearoyl-sn-glycero-3-phosphoglycerol, sodium salt (DSPG), pegylated, polyglycerylated phospholipids, and/or sterols containing PEG covalently attached to the lipid molecule (pegylated lipids) having a molecular weight from between approximately 350 to 6000 Da.

The non-pegylated phospholipids can preferably be used in a molar % from between approximately 2.5 to 35 molar%, preferably in molar % from between approximately 15 to 25 molar%, and most preferably approximately 20 molaro.

The pegylated lipids can preferably be used in a molar % from between approximately 1 to 10 molar%, preferably in molar % from between approximately 2 to 5 molar%, and most preferably approximately 5 molaro.

The sterols, for example, and preferably cholesterol, can preferably be used in a molar % from between approximately 0 to 50 molar%, preferably in molar % from between approximately 20 to 30 molaro.

In an embodiment, the liposomal preparation comprises a further excipient selected from the group of excipients comprising:
Amino acids in particular glycine or histidine;
Vitamins such as nicotinic acid amide;
Organic acids and their salts;
Saccharose (sucrose); and
Targeting moieties used for liposome targeting comprising in some non-limiting examples: transferrin, folic acid, and peptides.

There are many methods of liposome preparation. The simplest are the thin lipid film method, lyophilization of lipids from an organic solvent and hydration of the resulting lipid powder, and methods involving the injection of an organic phospholipid solution into an aqueous solution. Liposomes formed in this way are usually multi-layered and require reducing their size by using extrusion, sonication or high-pressure homogenization methods. Some methods involving mixing an aqueous drug solution with an ethanol solution of phospholipids or phospholipids with cholesterol and/or pegylated phospholipids allow obtaining small unilamellar liposomes or large unilamellar liposomes at once. The advantage of microfluidic methods is the possibility of obtaining liposomes of a specific size and high encapsulation or incorporation efficiency of hydrophilic or hydrophobic drugs at once. However, the disadvantage is the presence of a high concentration of ethanol in the formulation, ranging from 30 to 50% by volume. Long-circulating liposomes with a size of approx. 100 nm and below have, like other drug carrier particles, the ability to accumulate in inflamed tissues. This causes a significant increase in the accumulation of drugs in, for example, cancerous tumors, facilitating the treatment process. The smaller the liposomes, the more efficient the accumulation process. The accumulation process can be further enhanced by the use of certain molecules interacting with receptors or proteins present on the surface of tumor cells. The use of transferrin molecules, folic acid, and antibodies covalently attached to phospholipids that are components of liposomes may lead to increased accumulation of such liposomes in tumors or increase the degree of internalization of liposomes in cells. In the treatment of brain tumors, the use of transferrin and the small size of liposomes leads to a relatively efficient accumulation of liposomes in the brain. in other cases, liposome accumulation may be lower.

The process of encapsulating drugs in liposomes is of great importance for the fate of the drug in the body after administration of liposomes. In the case of hydrophilic drugs, very well soluble in water, passive methods of encapsulation LUV-type liposomes in the aqueous space are used. The rate of drug release depends more on the composition of the liposomes and less on the nature of the substance. In the case of lipophilic drugs with ionizable moieties, active encapsulation methods using a pH or ionic gradient are used. this usually leads to a very efficient encapsulation of a given drug and the rate of drug escape after intravenous administration depends on both the degree of hydrophobicity of the drug and the method that has been used. Knowing the properties of the drug and the method of encapsulation, one can theoretically prepare a drug formulation that will have optimal pharmacokinetic properties and release rate in the target tissue.

Multilamellar vesicles (MLV) liposomes of the invention may be prepared using various methods comprising, amongst others: thin film method, lyophilisation of lipids from organic solvent, injection of lipid solution in supercritical carbon dioxide to water, lipid solution spray drying, and/or dehydration-rehydration method.

Large unilamellar vesicles (LUV) liposomes of the invention may be prepared using various methods comprising, amongst others: alcohol injection methods, extrusion technique, high pressure homogenisation, sonication method, reverse phase evaporation, and/or microfluidic method.

Composition of liposomes according to the invention may comprise, amongst others: DPPC, SM, HSPC, DSPC, DPPG, DSPG, Chol, Pegylated lipids (phospholipids like DPPE-PEG, DSPE-PEG) (sterols like cholesterol-PEG), and/or polyglycerylated phospholipids).

In an embodiment, the liposomes comprise between approximately 30 to 99 molar% DPPC, SM, HSPC, and/or DSPC.

In an embodiment, the liposomes comprise between approximately 0.5 to 40 molar% DPPG and/or DSPG.

In an embodiment, the liposomes comprise between approximately 0 to 50 molar% cholesterol.

In an embodiment, the liposomes comprise between approximately 1 to 10 molar% pegylated lipids. The PEG molecular weight is preferably between approximately 350 to 6000.

In an embodiment, the liposomes comprise between approximately 1 to 20 molar% polyglycerylated phospholipids.

In an embodiment, the liposomes comprise polyglycerylated phospholipids glycerol units from between approximately 4 to 30.

In an embodiment, the liposomes comprise targeting moieties. The targeting moieties preferably comprise transferrin and/or folic acid attached to hydrophobic anchor. The hydrophobic anchor preferably comprises pegylated phospholipids and/or pegylated cholesterol.

In an embodiment, the liposomes comprise an external liposome buffer solution. The external liposome buffer solution preferably comprises excipients comprising organic acids and their salts. The organic acids and their salts preferably comprises one or more selected from the group comprising: citric acid, lactic acid, succinic acid, malic acid, tartrate acid, ascorbic acid, and acetic acid. The external liposome buffer solution preferably comprises excipients comprising sugars, wherein the sugars preferably comprise, amongst others: saccharose, trehalose, and/or glucose.

In an embodiment, the liposomes comprise an internal liposome buffer solution which comprises lomustine and cyclodextrin solution, wherein the cyclodextrin solution preferably comprises methyl-β-CD and/or hydroxy-propyl-β-CD in water. The internal liposome buffer solution preferably comprises excipients comprising organic acids and their salts. The organic acids and their salts preferably comprises one or more selected from the group comprising: citric acid, lactic acid, succinic acid, malic acid, tartrate acid, ascorbic acid, phytic acid, and acetic acid. The internal liposome buffer solution preferably comprises excipients comprising transit metal ions to form additional complexes with lomustine to stabilise drug encapsulation in the liposomes. The transit metal ions preferably comprise Cu^{+2,} Cu⁺¹.

In an embodiment, lomustine and cyclodextrin solution in water further comprises the excipient nicotinic acid in a range of between approximately 10 to 50 molaro.

### Example 1. Preparation of lomustine solution in methyl-β-CD.

Lomustine in an amount of 0.2 g was weighed together with 2.5 g methyl-β-CD. The substances were mixed together and then 20 mM citrate buffer pH 4.5 was added to make 10 ml of the solution. The solution was then mixed vigorously for 5 minutes using a vortex resulting in a yellowish solution of lomustine in methyl-β-CD.

### Example 2. Preparation of lomustine solution in hydroxyl propyl-β-CD.

Lomustine in an amount of 0.1 g was weighed together with 2.5 g hydroxyl propyl-β-CD. The substances were mixed together and then 20 mM citrate buffer pH 4.5 was added to make 10 ml of the solution. The solution was then mixed vigorously for 10 minutes using a vortex resulting in a yellowish solution of lomustine in methyl-β-CD.

### Example 3

0.4 g of lipids of the composition HSPC/DSPE-PEG 2000 (95:5, mol/mol) were dissolved in 10 mL of cyclohexane and frozen in liquid nitrogen. The resulting frozen solution was subsequently freeze-dried and the resulting dry lipid cake was suspended in 10 mL of lomustine solution in methyl-β-CD achieved following Example 1 at 64 °C. The multilamellar liposomes were then extruded through a 400 nm and then a 100 nm polycarbonate filter on a thermobarrel extruder in order to achieve large unilamellar liposomes. The non-encapsulated lomustine was removed by a gel filtration method using Sephadex G50 fine column equilibrated with 10% saccharose and 20 mM citrate buffer pH 4.5. The encapsulation efficiency of the lomustine was 3.5%, and the liposomes size was 120 nm.

### Example 4

0.4 g of lipids of the composition HSPC/DSPE-PEG 2000 (95:5, mol/mol) were dissolved in 10 mL of cyclohexane and frozen in liquid nitrogen. The resulting frozen solution was subsequently freeze-dried and the resulting dry lipid cake was suspended in 10 mL of lomustine solution in hydroxyl propyl-β-CD achieved following Example 2 at 64 °C. The multilamellar liposomes were then extruded through a 400 nm and then a 100 nm polycarbonate filter on a thermobarrel extruder in order to achieve large unilamellar liposomes. The non-encapsulated lomustine was removed by a gel filtration method using Sephadex G50 fine column equilibrated with 10% saccharose and 20 mM citrate buffer pH 4.5. The encapsulation efficiency of the lomustine was 3.9%, and the liposomes size was 115 nm.

### Example 5

0.4 g of lipids of the composition HSPC/DSPG/DSPE-PEG 2000 (75:20:5, mol/mol) were dissolved in 10 mL of cyclohexane with the addition of methanol and frozen in liquid nitrogen. The resulting frozen solution was subsequently freeze-dried and the resulting dry lipid cake was suspended in 10 mL of lomustine solution in methyl-β-CD achieved following Example 1 at 64 °C. The multilamellar liposomes were then extruded through a 400 nm and then a 100 nm polycarbonate filter on a thermobarrel extruder in order to achieve large unilamellar liposomes. The non-encapsulated lomustine was removed by a gel filtration method using Sephadex G50 fine column equilibrated with 10% saccharose and 20 mM citrate buffer pH 4.5. The encapsulation efficiency of the lomustine was 4.2%, and the liposomes size was 107 nm.

### Example 6

0.4 g of lipids of the composition HSPC/DSPG/DSPE-PEG 2000 (75:20:5, mol/mol) were dissolved in 10 mL of cyclohexane with the addition of methanol and frozen in liquid nitrogen. The resulting frozen solution was subsequently freeze-dried and the resulting dry lipid cake was suspended in 10 mL of lomustine solution in hydroxyl propyl-β-CD achieved following Example 1 at 64 °C. The multilamellar liposomes were then extruded through a 400 nm and then a100 nm polycarbonate filter on a thermobarrel extruder in order to achieve large unilamellar liposomes. The non-encapsulated lomustine was removed by a gel filtration method using Sephadex G50 fine column equilibrated with 10% saccharose and 20 mM citrate buffer pH 4.5. The encapsulation efficiency of the lomustine was 3,3%, and the liposomes size was 98nm.

### Example 7

0.4 g of lipids of the composition HSPC/Chol/DSPE-PEG 2000 (65:30:5, mol/mol) were dissolved in 10 mL of cyclohexane and frozen in liquid nitrogen. The resulting frozen solution was subsequently freeze-dried and the resulting dry lipid cake was suspended in 10 mL of lomustine solution in methyl-β-CD achieved following Example 1 at 64 °C. The multilamellar liposomes were then extruded through a 400 nm and then a 100 nm polycarbonate filter on a thermobarrel extruder in order to achieve large unilamellar liposomes. The non-encapsulated lomustine was removed by a gel filtration method using Sephadex G50 fine column equilibrated with 10% saccharose and 20 mM citrate buffer pH 4.5. The encapsulation efficiency of the lomustine was 3.7%, and the liposomes size was 117 nm.

### Example 8

0.4 g of lipids of the composition HSPC/Chol/DSPE-PEG 2000 (65:30:5, mol/mol) were dissolved in 10 mL of cyclohexane and frozen in liquid nitrogen. The resulting frozen solution was subsequently freeze-dried and the resulting dry lipid cake was suspended in 10 mL of lomustine solution in hydroxyl propyl-β-CD achieved following Example 1 at 64°C. The multilamellar liposomes were then extruded through a 400 nm and then a 100 nm polycarbonate filter on a thermobarrel extruder in order to achieve large unilamellar liposomes. The non-encapsulated lomustine was removed by a gel filtration method using Sephadex G50 fine column equilibrated with 10% saccharose and 20 mM citrate buffer pH 4.5. The encapsulation efficiency of the lomustine was 4.3%, and the liposomes size was 115 nm.

### Example 9

0.4 g of lipids of the composition SM/DSPE-PEG 2000 (95:5, mol/mol) were dissolved in 10 mL of cyclohexane and frozen in liquid nitrogen. The resulting frozen solution was subsequently freeze-dried and the resulting dry lipid cake was suspended in 10 mL of lomustine solution in methyl-β-CD achieved following Example 1 at 64 °C. The multilamellar liposomes were then extruded through a 400 nm and then a 100 nm polycarbonate filter on a thermobarrel extruder in order to achieve large unilamellar liposomes. The non-encapsulated lomustine was removed by a gel filtration method using Sephadex G50 fine column equilibrated with 10% saccharose and 20 mM citrate buffer pH 4.5. The encapsulation efficiency of the lomustine was 5.1%, and the liposomes size was 125 nm.

### Example 10

0.4 g of lipids of the composition SM/DSPE-PEG 2000 (95:5, mol/mol) were dissolved in 10 mL of cyclohexane and frozen in liquid nitrogen. The resulting frozen solution was subsequently freeze-dried and the resulting dry lipid cake was suspended in 10 mL of lomustine solution in hydroxyl propyl-β-CD achieved following Example 1 at 64 °C. The multilamellar liposomes were then extruded through a 400 nm and then a 100 nm polycarbonate filter on a thermobarrel extruder in order to achieve large unilamellar liposomes. The non-encapsulated lomustine was removed by a gel filtration method using Sephadex G50 fine column equilibrated with 10% saccharose and 20 mM citrate buffer pH 4.5. The encapsulation efficiency of the lomustine was 4.2%, and the liposomes size was 127 nm.

### Example 11

0.4 g of lipids of the composition HSPC/DSPE-PEG 2000 (95:5, mol/mol) were dissolved in 5 mL of ethanol then 20 mg of lomustine and 250 mg of methyl-β-CD were added. The resulting solution was evaporated using a rotatory evaporator and then dried under vacuum. The dry lipid film was resuspended in 10 mL of Milli-Q^{™} of water at 64 °C to form multilamellar liposomes. The multilamellar liposomes were then extruded through a 400 nm and then a 100 nm polycarbonate filter on a thermobarrel extruder in order to achieve large unilamellar liposomes. The non-encapsulated lomustine was removed by a gel filtration method using Sephadex G50 fine column equilibrated with 10% saccharose and 20 mM citrate buffer pH 4.5. The encapsulation efficiency of the lomustine was 6.1%, and the liposomes size was 103 nm.

### Example 12

0.4 g of lipids of the composition HSPC/DSPE-PEG 2000 (95:5, mol/mol) were dissolved in 5 mL of ethanol and then 10 mg of lomustine and 250 mg of hydroxyl propyl-β-CD were added. The resulting solution was evaporated on a rotatory evaporator and then dried under vacuum. The dry lipid film was resuspended in 10 mL of Milli-Q^{™} of water at 64 °C to form multilamellar liposomes. The multilamellar liposomes were then extruded through a 400 nm and then a 100 nm polycarbonate filter on a thermobarrel extruder in order to achieve large unilamellar liposomes. The non-encapsulated lomustine was removed by a gel filtration method using Sephadex G50 fine column equilibrated with 10% saccharose and 20 mM citrate buffer pH 4.5. The encapsulation efficiency of the lomustine was 5.3%, and the liposomes size was 108 nm.

### Example 13

The 4 ml of liposomes produced following the method described in Example 3 was placed in a 12 ml borosilate tube and frozen. The frozen liposomes were then lyophilised using a freeze-drying machine at -40 °C for 24 h. The resulting powder was rehydrated by the addition of 4 ml of MiliQ water. After the reconstitution, the size of the liposomes were 117 nm, while the PDI index changed from 0,097 to 0.132. The lomustine encapsulation efficiency was 82%.

Reference to cited material or information contained in the text should not be understood as a concession that the material or information was part of the common general knowledge or was known in Australia or any other country.

Each document, reference, patent application or patent cited in this text is expressly incorporated herein in their entirety by reference, which means that it should be read and considered by the reader as part of this text. That the document, reference, patent application, or patent cited in this text is not repeated in this text is merely for reasons for conciseness.

Optional embodiments of the present invention may also be said to broadly consist in the parts, elements and features referred to or indicated herein, individually or collectively, in any or all combinations of two or more of the parts, elements or features, and wherein specific integers are mentioned herein which have known equivalents in the art to which the invention relates, such known equivalents are deemed to be incorporated herein as if individually set forth.

It is to be appreciated that reference to "one example" or "an example" of the invention is not made in an exclusive sense. Accordingly, one example may exemplify certain aspects of the invention, whilst other aspects are exemplified in a different example. These examples are intended to assist the skilled person in performing the invention and are not intended to limit the overall scope of the invention in any way unless the context clearly indicates otherwise.

It is to be understood that the terminology employed above is for the purpose of description and should not be regarded as limiting. The described embodiment is intended to be illustrative of the invention, without limiting the scope thereof. The invention is capable of being practised with various modifications and additions as will readily occur to those skilled in the art.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs.

Various substantially and specifically practical and useful exemplary embodiments of the claimed subject matter are described herein, textually and/or graphically, including the best mode, if any, known to the inventors for carrying out the claimed subject matter.

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in the specification, individually or collectively and any and all combinations or any two or more of the steps or features.

The inventor(s) expects skilled artisans to employ such variations as appropriate, and the inventor(s) intends for the claimed subject matter to be practiced other than as specifically described herein. Accordingly, as permitted by law, the claimed subject matter includes and covers all equivalents of the claimed subject matter and all improvements to the claimed subject matter. Moreover, every combination of the above described elements, activities, and all possible variations thereof are encompassed by the claimed subject matter unless otherwise clearly indicated herein, clearly and specifically disclaimed, or otherwise clearly contradicted by context.

The present invention is not to be limited in scope by the specific embodiments described herein, which are intended for the purpose of exemplification only. Functionally equivalent products, compositions and methods are clearly within the scope of the invention as described herein.

The use of any and all examples, or exemplary language (e.g., "such as" or "for example") provided herein, is intended merely to better illuminate one or more embodiments and does not pose a limitation on the scope of any claimed subject matter unless otherwise stated. No language in the specification should be construed as indicating any non-claimed subject matter as essential to the practice of the claimed subject matter.

The use of words that indicate orientation or direction of travel is not to be considered limiting. Thus, words such as "front", "back", "rear", "side", "up", down", "upper", "lower", "top", "bottom", "forwards", "backwards", "towards", "distal", "proximal", "in", "out" and synonyms, antonyms and derivatives thereof have been selected for convenience only, unless the context indicates otherwise. The inventor(s) envisage that various exemplary embodiments of the claimed subject matter can be supplied in any particular orientation and the claimed subject matter is intended to include such orientations.

The use of the terms "a", "an", "said", "the", and/or similar referents in the context of describing various embodiments (especially in the context of the claimed subject matter) are to be construed to cover both the singular and the plural, unless otherwise indicated herein or clearly contradicted by context. The terms "including," "having," and "containing" are to be construed as open-ended terms (i.e., meaning "including, but not limited to,") unless otherwise noted.

Throughout the specification and claims, unless the context requires otherwise, the word "comprise" or variations such as "comprises" or "comprising", will be understood to imply the inclusion of a stated integer or group of integers but not the exclusion of any other integer or group of integers.

Moreover, when any number or range is described herein, unless clearly stated otherwise, that number or range is approximate. Recitation of ranges of values herein are merely intended to serve as a shorthand method of referring individually to each separate value falling within the range, unless otherwise indicated herein, and each separate value and each separate sub-range defined by such separate values is incorporated into the specification as if it were individually recited herein. For example, if a range of 1 to 10 is described, that range includes all values there between, such as for example, 1.1, 2.5, 3.335, 5, 6.179, 8.9999, etc., and includes all sub-ranges there between, such as for example, 1 to 3.65, 2.8 to 8.14, 1.93 to 9, etc.

Use of "approximately", "approximate", or variations thereof is to indicate that when used with respect to a stated number, amount, or value (or range - see previous paragraph), also includes numbers, amounts, or values within a close range to that number, amount, or value. Numbers, amounts, or values within that close range, for example, may include all values within 10% above and/or below the number, amount, or value (potentially to multiple decimal points). Otherwise, the normal dictionary definition for "approximately", "approximate", or variations thereof can be applied.

Accordingly, every portion (e.g., title, field, background, summary, description, abstract, drawing figure, etc.) of this application, other than the claims themselves, is to be regarded as illustrative in nature, and not as restrictive; and the scope of subject matter protected by any patent that issues based on this application is defined only by the claims of that patent.

In the figures, incorporated to illustrate features of a non-limiting example embodiment, like reference numerals are used to identify like parts throughout the figures.

While there are shown and described presently further embodiments of the application, it is to be distinctly understood that the application is not limited thereto but may be otherwise variously embodied and practiced within the scope of the following claims.

Below some of the above and further embodiments, in particular of the further aspects of the application, are further defined in the following list of embodiment items.

Item 1. A drug delivery solution for encapsulation within the aqueous core of liposomes, the drug delivery solution comprising:
an aqueous solution comprising at least one drug and cyclodextrins.

Item 2. A drug delivery solution according to item 1, wherein the aqueous solution comprises water and/or ethanol, methanol, or another organic solvent.

Item 3. A drug delivery solution according to item 1 or item 2, wherein the cyclodextrins comprise methyl-β-CD or hydroxy propyl-β-CD.

Item 4. A drug delivery solution according to item 3, wherein the methyl-β-CD or hydroxy propyl-β-CD comprises between approximately 1 and 45% by weight of the drug delivery solution, preferably between approximately 5 and 35% by weight of the drug delivery solution, or preferably approximately 25% by weight of the drug delivery solution.

Item 5. A drug delivery solution according to any one of the preceding items, wherein the at least one drug is mixed into and dissolves with the cyclodextrins in aqueous solution.

Item 6. A drug delivery solution according to any one of the preceding items, wherein the at least one drug comprises lomustine.

Item 7. A drug delivery solution according to item 6, wherein lomustine is added to methyl-β-CD and/or hydroxy propyl-β-CD and the compounds are mixed in 20 mM citrate buffer pH 4.5 to form the drug delivery solution.

Item 8. A drug delivery solution according to any one of the preceding items, wherein the concentration of lomustine in the drug delivery solution is between approximately 1 to 40 mg/ml, preferably between approximately 10 to 30 mg/ml, or preferably approximately 20 mg/ml.

Item 9. A drug delivery solution according to any one of the preceding items, wherein the amount of lomustine is between approximately 0.025 to 4% of the total weight of the drug delivery solution, preferably between approximately 0.5 to 1.5% of the total weight of the drug delivery solution when the cyclodextrin comprises hydroxy propyl-β-CD, or preferably between approximately 1 to 3% of the total weight of the drug delivery solution when the cyclodextrin comprises methyl-β-CD.

Item 10. A drug delivery solution according to any one of the preceding items, wherein the drug delivery solution is subject to size modification processes.

Item 11. A drug delivery solution according to item 10, wherein the size modification processes comprise one or more of extrusion, high pressure homogenisation, or sonication.

Item 12. A drug delivery solution according to any one of the preceding items, wherein lomustine is added to methyl-β-CD and the compounds are mixed in 20 mM citrate buffer pH 4.5 to form the drug delivery solution.

Item 13. A drug delivery solution according to any one of the preceding items, wherein lomustine is added to hydroxy propyl-β-CD and the compounds are mixed in 20 mM citrate buffer pH 4.5 to form the drug delivery solution.

Item 14. A method of forming liposomes comprising: combining a drug delivery solution according to any one of items 1 to 13 with phospholipids, lipids, and with or without sterols to form a liposome forming mixture;
drying to evaporate the liquid from the liposome forming mixture;
rehydrating the liposome forming mixture in water and excipients to form multilamellar liposomes.

Item 15. A method according to item 14, wherein the pH of the aqueous solution core of the liposomes is less than approximately pH 5.5, or preferably between approximately pH 4.0 to 5.0.

Item 16. A method according to item 14 or 15, wherein the phospholipids and any other lipids comprises between approximately 0.1 to 10% by weight (w/w) of the total mass of liposomes suspension, or preferably between approximately 1 to 5% by weight (w/w) of the total mass of liposomes suspension.

Item 17. A method according to any one of items 14 to 16, wherein the phospholipids comprise one or more selected from the group comprising: sphingomyelins (SM), dipalmitoylphosphatidylcholine (DPPC), distearoylphosphatidylcholine (DSPC), and hydrogenated soybean phospholipids (HSPC).

Item 18. A method according to any one of items 14 to 17, wherein the auxiliary phospholipids and lipids comprise one or more selected from the group comprising: 1,2-Dipalmitoyl-sn-glycero-3-phosphoglycerol, sodium salt (DPPG), 1,2-Distearoyl-sn-glycero-3-phosphoglycerol, sodium salt (DSPG), pegylated, polyglycerylated phospholipids, and/or sterols containing PEG covalently attached to the lipid molecule (pegylated lipids) having a molecular weight from between approximately 350 to 6000 Da.

Item 19. A method according to item 18, wherein the non-pegylated auxiliary phospholipids are used in a molar % from between approximately 2.5 to 35 molar%, preferably between approximately 15 to 25 molar%, or preferably in a molar % of approximately 20 molaro.

Item 20. A method according to item 18 or 19, wherein the pegylated lipids are used in a molar % from between approximately 1 to 10 molar%, preferably between approximately 2 to 5 molar%, or preferably in a molar % of approximately 5 molaro.

Item 21. A method according to any one of items 18 to 20, wherein the sterols comprise cholesterol and are used in a molar % from between approximately 0 to 50 molar%, preferably between approximately 20 to 30 molaro.

Item 22. A method according to any one of items 14 to 21, wherein the excipients comprise one or more from the group selected from: amino acids, vitamins, organic acids and their salts, saccharose, and targeting moieties used for liposome targeting.

Item 23. A method according to item 22, wherein the amino acids comprise glycine and/or histidine.

Item 24. A method according to item 22 or 23, wherein the vitamins comprise nicotinic acid amide.

Item 25. A method according to any one of items 22 to 24, wherein the targeting moieties comprise one or more of transferrin, folic acid, and peptides.

Item 26. A method of forming liposomes comprising:
dissolving a lipid composition in an organic solvent and then drying the dissolved lipids;
resuspending the lipids in a drug delivery solution according to any one of items 1 to 13 to form multilamellar liposomes;
extruding the multicellular liposomes to form large unilamellar liposomes; and
removing non-encapsulated drug by gel filtration.

Item 27. A method according to item 26, wherein the lipid composition comprises HSPC/DSPE-PEG 2000 (95:5, mol/mol).

Item 28. A method according to item 26, wherein the lipids composition comprises HSPC/DSPG/DSPE-PEG 2000 (75:20:5, mol/mol).

Item 29. A method according to item 26, wherein the lipids composition comprises HSPC/Chol/DSPE-PEG 2000 (65:30:5, mol/mol).

Item 30. A method according to item 26, wherein the lipids composition comprises SM/DSPE-PEG 2000 (95:5, mol/mol).

Item 31. A method according to item 26, wherein the lipids composition comprises SM/Chol (45:55, mol/mol).

Item 32. A method according to any one of items 26 to 31, wherein the organic solvent comprises one or more selected from the group comprising: cyclohexane, methanol, and ethanol.

Item 33. A method according to any one of items 26 to 32, wherein the drying comprises freeze drying.

Item 34. A method according to item 33, wherein the freeze drying comprises using liquid nitrogen.

Item 35. A method according to any one of items 26 to 32, wherein the drying comprises evaporation.

Item 36. A method according to item 35, wherein the evaporation uses a rotary evaporator and vacuum.

Item 37. A method according to any one of items 26 to 36, wherein the lipids are resuspended in the drug delivery solution at approximately 64 °C.

Item 38. A method according to any one of items 26 to 37, wherein the multicellular liposomes are extruded through a 400 nm and then 100 nm polycarbonate filter using a thermobarrel extruder.

Item 39. A method according to any one of items 26 to 38, wherein the non-encapsulated drug is removed by gel filtration using a Sephadex G50 fine column equilibrated with 10% saccharose and 20 mM citrate buffer pH 4.5.

Item 40. A method according to any one of items 26 to 39, wherein the size of the unilamellar liposomes was between approximately 90 to 130 nm.

Item 41. A method according to any one of items 26 to 40, wherein the drug encapsulation efficiency of the method was between approximately 3.0 to 6.5%.

Item 42. Liposomes produced by a method according to any one of items 14 to 41, that are lyophilised and then rehydrated in water.

Item 43. Liposomes according to item 42 that are lyophilised by freeze-drying at approximately 40 °C for 24 h.

Item 44. Liposomes according to item 42 or item 43, wherein the rehydrated liposomes are between approximately 105 to 125 nm in size, or preferably approximately 117 nm.

Item 45. Liposomes according to any one of items 42 to 44, wherein the rehydrated liposomes have a PDI Index of between approximately 0.1 to 0.2, or preferably approximately 0.132.

Item 46. Liposomes according to any one of items 42 to 45, wherein the liposomes have an encapsulation efficiency of between approximately 3.9-6.1%, or preferably between approximately 4-6%.

Item 47. Liposomes formed by a method according to any one of items 14 to 41.

Item 48. Liposomes formed by a method according to any one of items 14 to 41 for treating cancer.

Item 49. A method of treating a subject in need thereof with liposomes according to item 47.

Item 50. A method of treating cancer using liposomes formed by a method according to any one of items 14 to 41.

Item 51. Use of liposomes according to item 47 for administration to a subject in need thereof in the treatment of cancer.

Item 52. The use of lomustine in the manufacture of a medicament comprising liposomes according to item 47 for the therapeutic treatment of cancer.

Item 53. The methods and uses according to any one of items 49 to 52, wherein treating the subject comprises injecting the subject with the liposomes.

Item 54. The methods and uses according to any one of items 49 to 53, wherein the subject is injected intravenously.

Item 55. The methods and uses according to any one of items 49 to 54, wherein the subject is treated for cancer.

Item 56. The methods and uses according to any one of items 49 to 55, wherein the subject is treated for one or more selected from the group comprising: brain tumors, gliomas, glioblastoma, leukemia, lymphomas, Hodgkin's disease, non-Hodgin's lymphomas, lung cancer, blood cancers, malignant melanoma, breast carcinoma, bronchogenic carcinoma, renal cell carcinoma, carcinoma of the GI tract, and various solid tumors.

Item 57. Liposomes as formed by a method according to any one of items 14 to 41 for use in treating cancer.

Item 58. Liposomes as formed by a method according to any one of items 14 to 41, for use in a method of treating the human body by therapy.

Item 59. Use of a drug delivery solution according to items 1 to 13 in a composition comprising liposomes as formed by a method according to any one of items 14 to 41, for the treatment of cancer.

Item 60. Use of a drug delivery solution according to items 1 to 13 in a composition comprising liposomes as formed by a method according to any one of items 14 to 41, as a medicament for the treatment of cancer.

Item 61. Use of liposomes as formed by a method according to any one of items 14 to 41, for the treatment of cancer.

Item 62. Method of treatment of cancer using liposomes as formed by a method according to any one of items 14 to 41.

Item 63. Use of a drug delivery solution according to items 1 to 13 in a composition comprising liposomes as formed by a method according to any one of items 14 to 41, for the treatment of cancer or one or more selected from the group comprising: brain tumors, gliomas, glioblastoma, leukemia, lymphomas, Hodgkin's disease, non-Hodgin's lymphomas, lung cancer, blood cancers, malignant melanoma, breast carcinoma, bronchogenic carcinoma, renal cell carcinoma, carcinoma of the GI tract, and various solid tumors.

Item 64. Use according to item 63, wherein the treatment of cancer or one or more selected from the group comprising: brain tumors, gliomas, glioblastoma, leukemia, lymphomas, Hodgkin's disease, non-Hodgin's lymphomas, lung cancer, blood cancers, malignant melanoma, breast carcinoma, bronchogenic carcinoma, renal cell carcinoma, carcinoma of the GI tract, and various solid tumors, comprises injecting a subject in need thereof intravenously with the liposomes as formed by a method according to any one of items 14 to 41.

Item 65. Use according to item 63, wherein the treatment comprises injecting a subject with the liposomes.

Item 66. Use according to item 65, wherein the subject is injected intravenously.

Item 67. A drug delivery solution for encapsulation within the aqueous core of liposomes, the drug delivery solution comprising:
an aqueous solution comprising at least one drug and cyclodextrins;
wherein the cyclodextrins comprise methyl-β-CD or hydroxy propyl-β-CD;
wherein the at least one drug is mixed into and dissolves with the cyclodextrins in aqueous solution;
wherein the at least one drug comprises lomustine and the concentration of lomustine in the drug delivery solution is between approximately 1 to 40 mg/ml, preferably between
approximately 10 to 30 mg/ml, or preferably approximately 20 mg/ml; and
wherein the methyl-β-CD or hydroxy propyl-β-CD comprises between approximately 1 and 45% by weight of the drug delivery solution, preferably between approximately 5 and 35% by weight of the drug delivery solution, or preferably approximately 25% by weight of the drug delivery solution.

## Claims

1. A drug delivery solution for encapsulation within the aqueous core of liposomes, the drug delivery solution comprising:
an aqueous solution comprising at least one drug and cyclodextrins;
wherein the cyclodextrins comprise methyl-β-CD or hydroxy propyl-β-CD;
wherein the at least one drug is mixed into and dissolves with the cyclodextrins in aqueous solution;
wherein the at least one drug comprises lomustine and the concentration of lomustine in the drug delivery solution is between approximately 1 to 40 mg/ml, preferably between approximately 10 to 30 mg/ml, or preferably approximately 20 mg/ml; and
wherein the methyl-β-CD or hydroxy propyl-β-CD comprises between approximately 1 and 45% by weight of the drug delivery solution, preferably between approximately 5 and 350 by weight of the drug delivery solution, or preferably approximately 25% by weight of the drug delivery solution.

2. A drug delivery solution according to claim 1, wherein the aqueous solution comprises water and/or ethanol, methanol, or another organic solvent.

3. A drug delivery solution according to claim 1 or claim 2, wherein lomustine is added to methyl-β-CD and/or hydroxy propyl-β-CD and the compounds are mixed in 20 mM citrate buffer pH 4.5 to form the drug delivery solution.

4. A drug delivery solution according to any one of the preceding claims, wherein the amount of lomustine is between approximately 0.025 to 4% of the total weight of the drug delivery solution, preferably between approximately 0.5 to 1.5% of the total weight of the drug delivery solution when the cyclodextrin comprises hydroxy propyl-β-CD, or preferably between approximately 1 to 3% of the total weight of the drug delivery solution when the cyclodextrin comprises methyl-β-CD.

5. A method of forming liposomes comprising:
combining a drug delivery solution according to any one of claims 1 to 4 with phospholipids, lipids, and with or without sterols to form a liposome forming mixture;
drying to evaporate the liquid from the liposome forming mixture;
rehydrating the liposome forming mixture in water and excipients to form a multilamellar liposomes suspension.

6. A method according to claim 5, wherein the pH of the solution in the aqueous core of the liposomes is less than approximately pH 5.5, or preferably between approximately pH 4.0 to 5.0.

7. A method according to claim 5 or claim 6, wherein the phospholipids and any other lipids comprises between approximately 0.1 to 10% by weight (w/w) of the total mass of liposomes suspension, or preferably between approximately 1 to 5% by weight (w/w) of the total mass of liposomes suspension, and
wherein the aqueous solution comprises water and/or ethanol, methanol, or another organic solvent.

8. A method according to any one of claims 5 to 7, wherein the phospholipids comprise one or more selected from the group comprising: sphingomyelins (SM), dipalmitoylphosphatidylcholine (DPPC), distearoylphosphatidylcholine (DSPC), and hydrogenated soybean phospholipids (HSPC);
wherein the auxiliary phospholipids comprise one or more selected from the group comprising: 1,2-Dipalmitoyl-sn-glycero-3-phosphoglycerol, sodium salt (DPPG), 1,2-Distearoyl-sn-glycero-3-phosphoglycerol, sodium salt (DSPG), pegylated, polyglycerylated phospholipids, and/or sterols containing PEG covalently attached to the lipid molecule (pegylated lipids) having a molecular weight from between approximately 350 to 6000 Da;
wherein the non-pegylated auxiliary phospholipids are used in a molar % from between approximately 2.5 to 35 molar%, preferably between approximately 15 to 25 molar%, or preferably in a molar % of approximately 20 molaro;
wherein the pegylated lipids are used in a molar % from between approximately 1 to 10 molar%, preferably between approximately 2 to 5 molar%, or preferably in a molar % of approximately 5 molaro; and/or
wherein the sterols comprise cholesterol and are used in a molar % from between approximately 0 to 50 molar%, preferably between approximately 20 to 30 molar%.

9. A method of forming liposomes comprising:
dissolving a lipid composition in an organic solvent and then drying the dissolved lipids;
resuspending the lipids in a drug delivery solution according to any one of claims 1 to 4 to form multilamellar liposomes;
extruding the multicellular liposomes to form large unilamellar liposomes; and
removing non-encapsulated drug by gel filtration.

10. A method according to claim 9, wherein the lipid composition comprises HSPC/DSPE-PEG 2000 (95:5, mol/mol);
wherein the lipids composition comprises HSPC/DSPG/DSPE-PEG 2000 (75:20:5, mol/mol);
wherein the lipids composition comprises HSPC/Chol/DSPE-PEG 2000 (65:30:5, mol/mol);
wherein the lipids composition comprises SM/DSPE-PEG 2000 (95:5, mol/mol); or
wherein the lipids composition comprises SM/Chol (45:55, mol/mol.

11. Liposomes formed by a method according to any one of claims 5 to 10.

12. Use of a drug delivery solution according to claims 1 to 4 in a composition comprising liposomes as formed by a method according to any one of claims 5 to 10, for the treatment of cancer or one or more selected from the group comprising: brain tumors, gliomas, glioblastoma, leukemia, lymphomas, Hodgkin's disease, non-Hodgin's lymphomas, lung cancer, blood cancers, malignant melanoma, breast carcinoma, bronchogenic carcinoma, renal cell carcinoma, carcinoma of the GI tract, and various solid tumors.

13. Use according to claim 12, wherein the treatment of cancer or one or more selected from the group comprising: brain tumors, gliomas, glioblastoma, leukemia, lymphomas, Hodgkin's disease, non-Hodgin's lymphomas, lung cancer, blood cancers, malignant melanoma, breast carcinoma, bronchogenic carcinoma, renal cell carcinoma, carcinoma of the GI tract, and various solid tumors, comprises injecting a subject in need thereof intravenously with the liposomes as formed by a method according to any one of claims 5 to 10.

14. Use according to claim 13, wherein the treatment comprises injecting a subject with the liposomes.

15. Use according to claim 14, wherein the subject is injected intravenously.
